# EUROPÄISCHE PATENTSCHRIFT

(11) **EP 1 290 991 B1**
(45) Veröffentlichungstag und Bekanntmachung des Hinweises auf die Patenterteilung: **31.10.2007**
(21) Anmeldenummer: 01810866.2
(22) Anmeldetag: 10.09.2001
(51) Int. Cl.: A61F 2/30

(54) **Femurhalsprothese**
Femoral prosthesis with a neck
Prothèse fémorale à col

(43) Veröffentlichungstag der Anmeldung: 12.03.2003
(73) Patentinhaber: Zimmer GmbH, 8404 Winterthur (CH)
(72) Erfinder: LeGros, Brian N., Chatham, Ontario N7M 1Z8 (CA); Fröhlich, Markus, 8362 Balterswil (CH); Wyss, Urs P., Dr., 8352 Räterschen (CH); Ploeg, Heidy-Lynn, Ottawa, Ontario K1J6J3 (CA)
(74) Vertreter: Manitz, Finsterwald & Partner GbR

(56) Entgegenhaltungen:
- EP-A- 0 019 044
- EP-A- 0 420 435
- DE-A- 2 621 123
- US-A- 6 120 544

## Beschreibung

Die Erfindung handelt von einer Femurhalsprothese mit einem kugelförmigen Kopf, durch den ein Kugelmittelpunkt definiert ist, und mit einem Schaft, welcher sich von der Höhe des Kugelmittelpunktes entlang einer Mittellinie über eine Länge L bis zu einem distalen Ende erstreckt.

Eine Schenkelhalsendoprothese mit einer kurzen Einbaulänge und einem abgebogenen, sich sprunghaft gegen caudal verjüngenden Schaftende ist in der Patentschrift U.S. 6,120,544 gezeigt. Schaftende und oberer Bereich der zementlos einsetzbaren Prothese sind mit einer dreidimensionalen Raumnetzstruktur belegt, um ein besseres Einwachsen von Knochenmaterial zu ermöglichen. Als Werkstoffe sind wie bei einer Druckscheibenprothese die für Implantate üblichen Metalle vorgesehen. Eine derartige Konstruktion hat im Vergleich zum Knochenmaterial, in das sie eingebettet wird, eine wesentlich grössere Steifigkeit an der Grenzfläche zum Knochenmaterial, die zwangsläufig zu ungewollt grossen Schubspannungen in diesen Grenzflächen führt.

Aufgabe der vorliegenden Erfindung ist es, einen schonenden Übergang der Kräfte von der Prothese auf das sie umgebende Knochenmaterial zu bilden. Diese Aufgabe wird dadurch erfüllt, dass der Schaft einen Elastizitätsmodul E zwischen 10 GPa und 60 GPa aufweist, dass die Länge L zwischen 50 mm und 150 mm liegt, und dass die aus dem Produkt von Elastizitätsmodul E und Länge L gebildete Ersatzsteifigkeit S = E · L zwischen 0,5 · 10⁹ und 9 · 10⁹ [Newton/Meter] liegt.

Durch die Angleichung des Elastizitätsmoduls E der Prothese an den des sie umgebenden Knochenmaterials unter Berücksichtigung einer Länge L der Prothese, die zwischen 150 mm und 50 mm liegt, erfahren grosse Bereiche des oberen Femurknochens eine ähnliche Belastung wie unter natürlichen Bedingungen. Der Schaft verhält sich ähnlich wie das Knochenmaterial selbst. Gleichzeitig wird der Verlust an Knochenmaterial klein gehalten.

Die abhängigen Ansprüche 2 bis 15 stellen vorteilhafte Weiterbildungen der Erfindung dar.

So ist es vorteilhaft, den Schaft mit einer in der Frontalebene liegenden Krümmung der Mittellinie auszuführen, die in Richtung distal zur Femurachse enger wird, um in die Femurachse einzumünden oder mit einem Knick in die Femurachse überzugehen, damit eine gute Annäherung an die natürliche Form des Femurknochens stattfindet. Diese wird noch verbessert, wenn der Schaft von proximal nach distal abnehmende elliptische Querschnitte aufweist, die längs der Mittellinie über einen Krümmungswinkel α von beispielsweise 45° um einen Verdrehwinkel ε ≤ 8° verschwenkt zueinander sind.

Im weiteren kann die Mantelfläche des Schaftes an den Orten mit grosser Belastung, d.h. unterhalb des Kopfes medial am Schaft eine erste Kontaktfläche aufweisen, die nah an die Kortikalis herangeführt ist und parallel zu dieser verläuft, sowie im Femurmarkraum lateral am Schaft eine zweite Kontaktfläche zur Kortikalis des Femurknochens aufweisen, um ein Kippmoment des Schaftes an den Knochen zu übergeben. Diese Kontaktflächen, welche unmittelbar für den Kontakt zur Kortikalis vorgesehen sind, haben eine Fläche von 150 bis 450 mm², um die spezifische Pressung niedrig zu halten. Eine weitere Möglichkeit für die Aufnahme von Belastungskräften besteht in einem unmittelbar an den Kopf angrenzenden Kragen der auf dem reserzierten Femurhals aufsitzt. Der Kragen steht dabei nicht notwendigerweise senkrecht zur Mittellinie des Schaftes, sondern die Ebene in der der Kragen liegt ist bezüglich der durch ihre Lage übernommenen Kräfte so optimiert, dass dieser Ebene eine bestimmte Lage zugeordnet ist, derart, dass diese Ebene mit ihrer Normalen in der Projektion auf die Frontalebene zu einer gegen medial verlaufenden, horizontalen X-Achse einen Winkel β = 87° ± 3°, in der Projektion auf die Sagittalebene mit der vertikalen Z-Achse einen Winkel γ = 15° ± 3° und in der Projektion auf die Transversalebene mit der gegen medial horizontalen X-Achse einen Winkel δ = 13° ± 3° bildet.

Als Schaftmaterial mit einem Elastizitätsmodul zwischen 10 GPa und 60 GPa kommen praktisch nur nichtmetallische Werkstoffe, also Kunststoffe oder Kunststoffe die durch Fasermaterial oder Füllstoffe verstärkt sind, in Frage. Reines PEEK kann in seinem E-Modul von 3.5 GPa durch Zumischen solcher Fasermaterialien und Füllstoffe bis auf einen Wert von 150 GPa gebracht werden. Die nachstehende Tabelle zählt beispielhaft mögliche Kunststoffe, ihre Zugfestigkeit und ihren E-Modul bezüglich Zugbelastung auf.

| Material | Zugfestigkeit (MPa) | E-Modul (GPa) |
|---|---|---|
| PEEK (Polyetheretherketon) | 93,8 | 3,5 |
| PEI (Polyetherimid) | 105 | 3,0 |
| PET (Polyethylenerephthalat) | 159 | 8,96 |
| PPS (Polyphenylensulfid) | 138 | 11,7 |
| PAEK (Polyaryletherketon) | 121 | 9,0 |

Diese können durch einen der nachfolgenden Stoffe in Form von Fasern oder Pulver verstärkt sein, um den E-Modul auf einen gewünschten Wert zu bringen:
Glas, Glasfasern, Kohlenstoff, Kohle resp. Graphitfasern, organische Verbindungen, Bor, Siliziumkarbid oder Keramik.

Fasermaterialien können aber auch als Gewebe oder Geflecht im Schaftkörper eingebettet sein.

Da der Kopf der Prothese als Lager anderen Kriterien genügen muss, kann es sinnvoll sein, den Kopf aus einem anderen Material beispielsweise als hohle Metallkugel oder als Keramikkugel auszuführen. Dabei gibt es entweder die Möglichkeit, dass Teile vom Kopf den Schaft durchdringen oder dass Teile vom Schaft den Kopf durchdringen, um eine gute Verbindung zwischen beiden Komponenten herzustellen.

Im folgenden wird die Erfindung anhand von Ausführungsbeispielen beschrieben. Es zeigen:
- Fig. 1: Schematisch eine Ansicht von anterior auf eine erfindungsgemässe, in der Frontalebene abgebildete Prothese in einem Femurknochen;
- Fig. 2: schematisch die Prothese der Fig. 1 von proximal gesehen;
- Fig. 3: schematisch die Prothese der Fig. 1 von lateral gesehen;
- Fig. 4: schematisch ein weiteres Ausführungsbeispiel einer Femurhalsprothese ohne Kragen von anterior gesehen;
- Fig. 5: schematisch die Prothese von Fig. 4 von proximal gesehen;
- Fig. 6: schematisch die Prothese von Fig. 4 von medial gesehen;
- Fig. 7: schematisch einen Schnitt VII-VII in Fig. 4;
- Fig. 8: schematisch einen Schnitt VIII-VIII in Fig. 4;
- Fig. 9: schematisch eine Ansicht von anterior und lateral auf eine Prothese analog zu Fig. 1, auf der erste und zweite Kontaktflächen eingezeichnet sind;
- Fig. 10: schematisch in der Frontalebene von anterior die bevorzugte Winkellage für die Ebene des Kragens einer linken Femurhalsprothese mit Kragenauflage, dargestellt durch eine Normale, die durch den Mittelpunkt eines natürlichen Femurkopfes geht;
- Fig. 11: schematisch die Normale von Fig. 10 in ihrer Projektion auf eine sagittale Ebene von lateral gesehen;
- Fig. 12: schematisch die Normale von Fig. 10 in ihrer Projektion auf eine transversale Ebene von proximal gesehen;
- Fig. 13: schematisch einen Längsschnitt in der Frontalebene durch eine Femurkopfprothese, bei der Kopf und Schaft ein gleiches Polymer aufweisen;
- Fig. 14: schematisch einen Längsschnitt durch eine Prothese analog zu Fig. 13, bei welcher die Lagerfläche des Kopfes durch eine metallische Hohlkugel gebildet ist;
- Fig. 15: schematisch einen Längsschnitt durch eine Prothese analog zu Fig. 13, bei welcher der Schaft zur Verankerung in den kugelförmigen Kopf hineinragt;
- Fig. 16: schematisch einen Längsschnitt durch eine Prothese analog zu Fig. 13, bei welcher der kugelförmige Kopf zur Verankerung mit einer Verlängerung in den Schaft hineinragt; und
- Fig. 17: schematisch eine Grafik in der E-Modul und Prothesenlänge L sowie die mit der Erfindung bevorzugten Bereiche aufgetragen sind.

In den Figuren sind gleiche Hinweiszeichen für gleiche Funktionen verwendet.

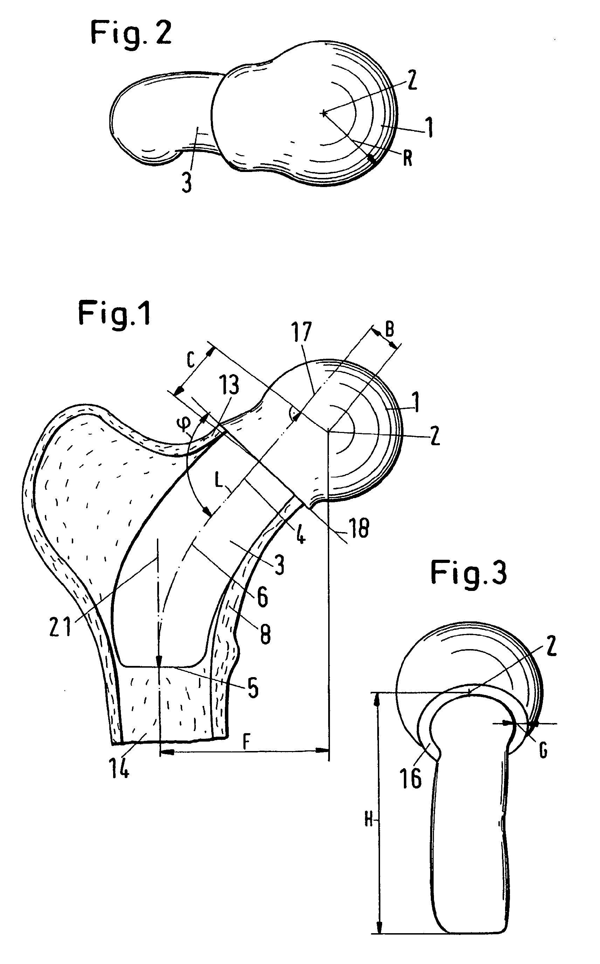

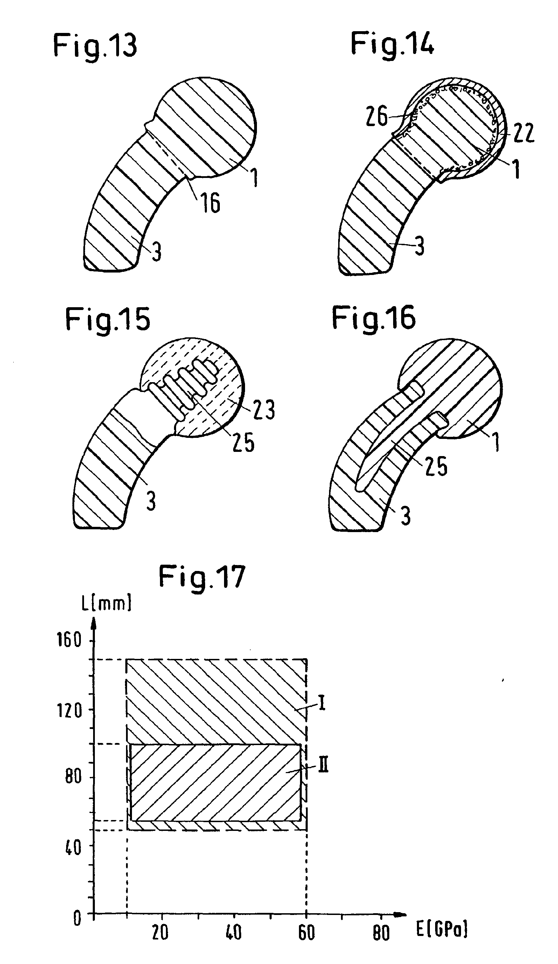

In einem Anwendungsbeispiel Figuren 1, 2 und 3 ist die Geometrie einer erfindungsgemässen Schenkelhalsprothese gezeigt, deren distales Ende 5 auf der Höhe vom kleinen Trochanter endet. Gegenüber der Längsachse 21 des Femurknochens 8 besitzt der Mittelpunkt 2 des Femurkopfes 1 eine Auslage F von 45 mm, die zwischen 30 bis 55 mm betragen kann. Die Mittellinie 4 vom Schaft 3 geht von der Femurachse 21 mit einer Krümmung 6 in den Femurhals 13 über. Der Schaft geht in einen Kragen 16 über, der sich auf einer durch die Resektionsfläche gebildeten Ebene 18 abstützt. Der Kugelmittelpunkt 2 ist gegenüber einer Verlängerung 17 der Mittellinie um einen Abstand B von 7 mm, welcher zwischen 3 und 12 mm betragen kann, gegen medial verschoben und um einen Abstand C von 18 mm, der zwischen 12 bis 26 mm betragen kann, gegen proximal gelegt. Der Radius R der Kugel, welcher 13 bis 30 mm betragen kann, wurde für dieses Beispiel auf 24 mm festgelegt. Das Schaftende 5 hat einen senkrechten Abstand H von 65 mm, der zwischen 50 und 85 mm betragen kann zum Kugelmittelpunkt 2. Die Auflagefläche des Kragens 16 ist wie die Ebene 18 in der Frontalebene um einen Winkel ϕ von 100° zur Mittellinie 4 geneigt. Der Kragen 16 hat eine Breite G von 3,5 mm, die von Null bis 6 mm betragen kann. Die Länge L des Schaftes ergibt sich entlang der Mittellinie 4 und deren Verlängerung 17 vom Schaftende 5 bis zur Höhe des Kugelmittelpunktes 2. Der E-Modul für diesen Schaft (siehe Grafik Fig. 17) kann zwischen 10 und 60 GPa (Giga Pascal) liegen. Der Schaft selbst hat eine elliptische Form der Querschnitte, die in distaler Richtung abnehmen und kann längs seiner Mittellinie 4 eine Verdrehung um einen Winkel ε ≤ 8° aufweisen, wie sie im nachfolgenden Ausführungsbeispiel beschrieben sind.

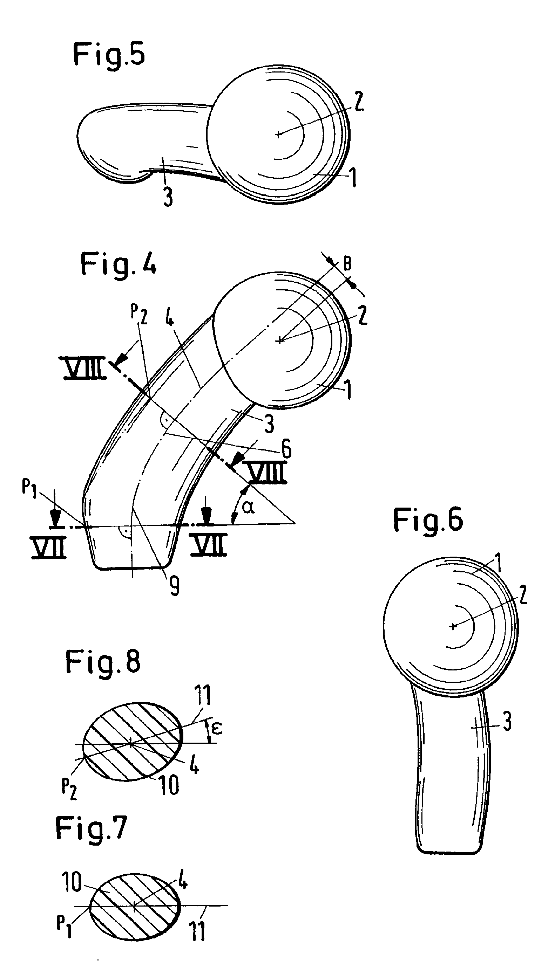

In einem weiteren Ausführungsbeispiel Figuren 4, 5, 6, 7 und 8 ist eine kragenlose Schenkelhalsprothese gezeigt, deren Kugelmittelpunkt 2 ebenfalls um einen Abstand B von der verlängerten Mittellinie 4 gegen medial verschoben ist. Die Mittellinie 4 geht in einer leichten Krümmung 6 auf das distale Ende zu und macht einen leichten Knick 9 für die Einmündung in die Richtung der Femurachse. Gemäss Fig. 7 besitzt das distale Ende einen elliptischen Querschnitt dessen grosse Achse 11 in der Frontalebene liegt und lateral durch einen Punkt P₁ geht. Wenn man sich in Fig. 4 aus diesem Querschnitt längs der gekrümmten Mittellinie 4 um einen Winkel α von 45° aufwärts bewegt, besteht dort wieder ein elliptischer, etwas grösserer Querschnitt (Fig. 8) dessen grosse Achse 11 um einen Winkel ε ≤ 8° verdreht ist, sodass ein zu Punkt P₁ korrespondierender Punkt P₂ auf der grossen Achse 11 mehr gegen anterior zu liegen kommt. Die Krümmung des Schaftes besteht nicht nur in der Frontalebene und es ergibt sich eine Anteversion für den Kugelkopf 1. Der E-Modul für den Schaft liegt ebenfalls zwischen 10 und 60 GPa.

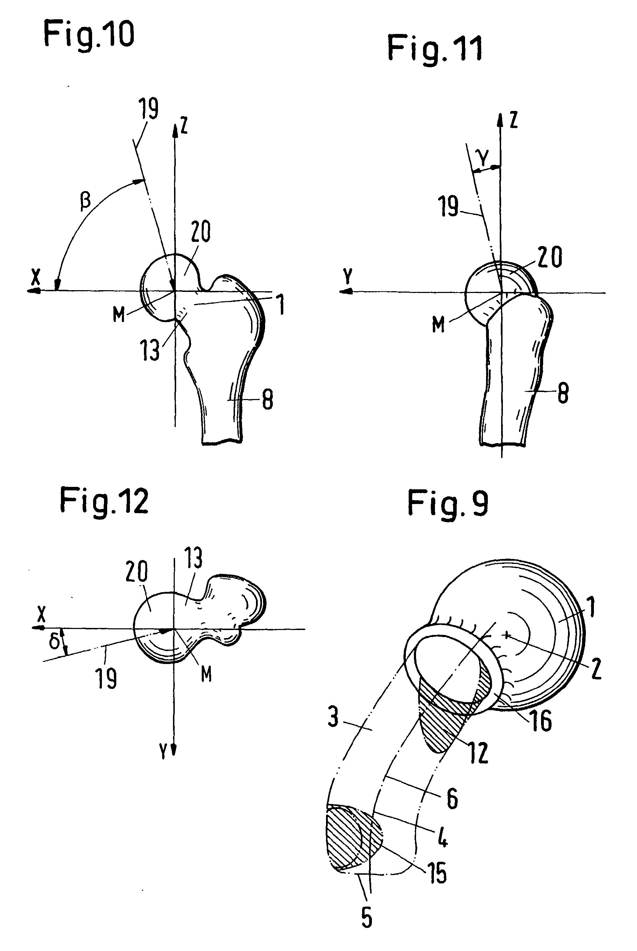

In Fig. 9 ist mit Phantomlinien die räumliche Lage einer ersten Kontaktfläche 12 und einer zweiten Kontaktfläche 15 angedeutet, welche besonders nah an der Kortikalis oder an dieser anliegend vorgesehen sind. Das Knochenbett wird in diesen Bereichen besonders sorgfältig vorbereitet. Die erste Kontaktfläche 12 liegt proximal angrenzend an den Kragen 16 gegen medial und bildet abgewickelt annähernd eine Dreieckfläche. Die zweite Kontaktfläche 15 liegt am distalen Schaftende gegen lateral angeordnet und hat die Form eines Pflasters, welches den Schaft nicht ganz auf 180° umschliesst. Es ist denkbar, den E-Modul im Bereich dieser Kontaktflächen etwas höher zu halten als an der restlichen Schaftoberfläche. Die Kontaktflächen 12, 15 können eine Ausdehnung von 150 bis 450 mm² aufweisen.

Das Material des Schaftes können Kunststoffe sein, wie sie in der vorausgehenden Tabelle aufgeführt sind, die durch Pulver oder Fasern eines anderen Materials in ihrem E-Modul heraufgesetzt sind. Für eine derartige Lösung ist interessant, dass auch pulverförmige Kunststoffe, die beispielsweise einen höheren Schmelzpunkt als das Grundmaterial aber einen etwa gleichen E-Modul besitzen, den Gesamt-E-Modul heraufsetzen können, wenn sie dispers verteilt sind. In Fig. 13 ist eine Schenkelhalsprothese gezeigt, deren Schaft 3 und Kopf 1 einstückig aus dem gleichen Material, beispielsweise aus Kunststoff mit pulverförmigen Füllstoffen gefertigt ist. Die eigentliche Lagerfläche am Kugelkopf ist mit einer Schutzschicht bedeckt, damit keine Füllstoffe direkt in Oberfläche hineinragen. Die Herstellung kann durch Thermoplast-Spritzen und Nacharbeiten am Kugelkopf erfolgen.

Bei der Schenkelhalsprothese in Fig. 14 ist eine vorgefertigte kugelförmige Kappe 22, beispielsweise aus Metall innen mit einer offenporigen Schicht 26 belegt und nachträglich mit einem mit Füllstoffen beladenen Thermoplast ausgespritzt worden, der gleichzeitig auch den Schaft bildet.

Bei der Schenkelhalsprothese in Fig. 15 besteht der Kugelkopf 1 aus einer Keramikkugel 23 mit einer Kavität, in die der Schaft 3 mit einem verlängernden Zapfen 25 hineinragt. Die Kontaktfläche zwischen Zapfen 25 und der Keramikkugel 23 ist zur Sicherung mit Vor- und Rücksprüngen versehen.

Bei der Schenkelhalsprothese in Fig. 16 besteht der Kopf 1 ebenfalls aus einem anderen Material mit einem höheren E-Modul als ihn der Schaft 3 aufweist. In den Schaft 3 ragt vom Kopf 1 her ein Zapfen 25 entlang der Mittelinie in den Schaft hinein um eine bessere Kraftübertragung zwischen Kopf und Schaft zu bewirken.

In Fig. 17 sind zwei Bereiche für die Erfindung aufgezeichnet. Ein erster Bereich für Schenkelhalsprothesen mit einer Länge L zwischen 50 und 150 mm und mit einem E-Modul zwischen 10 und 60 GPa ergibt Ersatzsteifigkeiten S = E · L, die zwischen 0,5 · 10⁹ und 9 · 10⁹ [Newton/Meter] liegen. Ein zweiter bevorzugter Bereich für mehrheitlich kurze Schenkelhalsprothesen mit einer Länge L zwischen 55 und 100 mm und mit einem E-Modul zwischen 10 und 60 GPa ergibt Ersatzsteifigkeiten zwischen 0,55 · 109 und 6 · 10⁹ [Newton/Meter].

In den Figuren 10, 11 und 12 zeigt die Normale 19 zur Resektionsebene, welche durch den Mittelpunkt M eines natürlichen und zu ersetzenden Femurkopfes 20 gezeichnet ist, wie ein Resektionsschnitt vorgenommen werden kann, um einen als günstig für die Kraftübertragung angesehenen Kragen aufzunehmen. Die Normale 19 steht in der Frontalebene (Fig. 10) in einem Winkel β von 87° ± 3° zur X-Achse, in der Sagitalebene (Fig. 11) in einem Winkel γ von 15° ± 3° zu der Z-Achse und in der Transversalebene (Fig. 12) in einem Winkel δ von 13° ± 3° zur X-Achse. Zur Auffindung der richtigen Lage für den Resektionsschnitt kann zunächst mit einer Vorbohrung durch den Femurkopf 20 die Lage der Normalen 19 festgelegt werden und anschliessend am Femurhals die Höhe für einen Resektionsschnitt senkrecht zur kenntlich gemachten Normalen 19 bestimmt werden.

Die Achsen X, Y, Z sind in ihrer genauen Lage zum Femurknochen nach G. Bergmann definiert [Habilitationsschrift Freie Universität Berlin 1994; Dr. Köster, Berlin 1997]:

"Die nach vorn gekrümmte Femurmittellinie tritt distal zwischen den Kondylen an einem Punkt aus dem Knochen aus. Im proximalen Femur schneiden sich Femurmittellinie und Schenkelhalsachse annähernd in einem weiteren Punkt. Durch diese beiden Punkte wird eine Gerade gelegt, welche als Femurlängsachse oder Femurachse bezeichnet wird. Sie ist gleichzeitig die nach proximal orientierte +z-Achse des Koordinatensystems. Durch den Austrittspunkt im Kondylenbereich wird eine Parallele zu Kniegelenkachse gelegt. Hierbei wird die Knieachse durch die Mittelpunkte der in der Sagittalebene annähernd halbkreisförmigen dorsalen Kondylenanteile definiert.

Verschobene Kniegelenkachse und Femurachse spannen die Frontalebene des Femurs auf. In dieser Ebene liegt senkrecht zu +z die nach medial gerichtete +x-Achse des Koordinatensystems, welche nicht genau parallel zu Knieachse ist. Senkrecht zu +x und +z wird die +y-Achse festgelegt. Ihre positive Richtung weist für das linke und rechte Hüftgelenk nach vorne. Links liegt somit ein rechtdrehendes, orthogonales Koordinatensystem vor, auf der rechten Seite dagegen ein linksdrehendes. Somit liegt (x y) die Transversalebene des Knochens fest und (y z) definiert die Sagittalebene".

## Patentansprüche

1. Femurhalsprothese mit einem kugelförmigen Kopf (1), durch den ein Kugelmittelpunkt (2) definiert ist, und mit einem Schaft (3), welcher sich von der Höhe des Kugelmittelpunktes (2) entlang einer Mittellinie (4) über eine Länge L bis zu einem distalen Ende (5) erstreckt, **dadurch gekennzeichnet, dass** der Schaft einen Elastizitätsmodul E zwischen 10 GPa und 60 GPa aufweist, dass die Länge L zwischen 50 mm und 150 mm liegt, und dass die aus dem Produkt von Elastizitätsmodul E und Länge L gebildete Ersatzsteifigkeit S = E ·L zwischen 0,5 · 10⁹ und 9 · 10⁹ [Newton/Meter] liegt.

2. Femurhalsprothese nach Anspruch 1, **dadurch gekennzeichnet, dass** die Mittellinie (4) des Schaftes (3) eine Krümmung (6) aufweist.

3. Femurhalsprothese nach einem der Ansprüche 1 oder 2, **dadurch gekennzeichnet, dass** der Schaft (3) an seinem Ende (5) gegen abwärts in der Richtung der Längsachse (7) eines Femurknochens (8) einen Knick (9) aufweist.

4. Femurhalsprothese nach einem der Ansprüche 1 bis 3, **dadurch gekennzeichnet, dass** der Schaft (3) senkrecht zur Mittellinie (4) bis zu seinem distalen Ende (5) elliptische Querschnitte (10) aufweist, deren Fläche mit der Annäherung an distal abnimmt.

5. Femurhalsprothese nach Anspruch 4, **dadurch gekennzeichnet, dass** die grossen Achsen (11) der elliptischen Querschnitte (10) mit der Verschiebung von proximal gegen distal eine Drehung um einen Verdrehwinkel ε ≤ 8° durchführen.

6. Femurhalsprothese nach einem der Ansprüche 1 bis 5, **dadurch gekennzeichnet, dass** unterhalb des Kopfes (1) medial am Schaft (3) eine in der Abwicklung ungefähr als Dreieck ausgebildete erste Kontaktfläche (12) für die Kortikalis eines reserzierten Femurhalses (13) vorgesehen ist und dass für den Bereich des Femurmarkraumes (14) lateral am Schaft (3) eine zweite Kontaktfläche (15) für die Kortikalis des Femurknochens (8) vorgesehen ist, um ein Kippmoment des Schaftes (3) an den Knochen (13, 8) zu übergeben.

7. Femurkopfprothese nach Anspruch 6, **dadurch gekennzeichnet, dass** die erste und zweite Kontaktfläche (12, 15) jeweils einer Fläche von 150 mm² bis 450 mm² entsprechen.

8. Femurkopfprothese nach einem der Ansprüche 1 bis 7, **dadurch gekennzeichnet, dass** der Schaft (3) zum Kopf (1) hin einen Kragen (16) aufweist.

9. Femurkopfprothese nach Anspruch 8, **dadurch gekennzeichnet, dass** der Kragen (16) in einer Ebene (18) liegt, der eine bestimmte Lage zugeordnet ist, derart, dass die Ebene (18) mit ihrer Normalen (19) in der Projektion auf die Frontalebene zu einer gegen medial verlaufenden, horizontalen X-Achse einen Winkel α = 87°, in der Projektion auf die Sagittalebene mit der vertikalen Z-Achse einen Winkel β = 15° und in der Projektion auf die Transversalebene mit der gegen medial verlaufenden, horizontalen X-Achse einen Winkel γ = 13° bildet, wobei die Winkelangaben um plus/minus 3° streuen dürfen.

10. Femurkopfprothese nach einem der Ansprüche 1 bis 9, **dadurch gekennzeichnet, dass** Kopf (1) und Schaft (3) aus unterschiedlichen Werkstoffen bestehen.

11. Femurkopfprothese nach Anspruch 10, **dadurch gekennzeichnet, dass** jeweils Kopf (1) und Schaft (3) aus einem nicht-metallischen Werkstoff bestehen.

12. Femurkopfprothese nach Anspruch 11, **dadurch gekennzeichnet, dass** der Kopf (1) aus einem keramischen Werkstoff besteht.

13. Femurkopfprothese nach Anspruch 10, **dadurch gekennzeichnet, dass** der Kopf (1) als hohle Metallkugel ausgeführt ist, in welche ein Schaft (3) aus Kunststoff eingeformt ist.

14. Femurkopfprothese nach Anspruch 10, **dadurch gekennzeichnet, dass** der Kopf (1) eine Verlängerung (17) aufweist, an welche ein Schaft (3) angeformt ist.

15. Femurkopfprothese nach einem der vorangehenden Ansprüche, **dadurch gekennzeichnet, dass** der Schaft (3) durch Fasermaterial oder Füllstoffe verstärkt ist, um den E-Modul auf einen gewünschten Wert zu bringen.

## Claims

1. A femoral neck prosthesis having a spherical shaped head (1), by which a centre (2) of a sphere is defined, and having a stem (3) which extends from the level of the centre (2) of the sphere along a centre line (4) over a length L up to a distal end (5), **characterised in that** the stem has a modulus of elasticity E of between 10 GPa and 60 GPa; **in that** the length L lies between 50 mm and 150 mm; and **in that** the equivalent stiffness S = E · L formed from the product of the modulus of elasticity E and the length L lies between 0.5 · 10⁹ and 9 · 10⁹ [Newton/metre].

2. A femoral neck prosthesis in accordance with claim 1, **characterised in that** the centre line (4) of the stem (3) has a curvature (6).

3. A femoral neck prosthesis in accordance with one of the claims 1 or 2, **characterised in that** the stem (3) has a downward kink (9) at its end (5) in the direction of the longitudinal axis (7) of a femur (8).

4. A femoral neck prosthesis in accordance with any one of the claims 1 to 3, **characterised in that** the stem (3) has elliptical cross-sections (10) perpendicular to the centre line (4) up to its distal end (5) whose areaurface reduces as they approach distal.

5. A femoral neck prosthesis in accordance with claim 4, **characterised in that** the large axes (11) of the elliptical cross-sections (10) perform a rotation about a rotational angle of ε ≤ 8° with the shift from proximal toward distal.

6. A femoral neck prosthesis in accordance with any one of the claims 1 to 5, **characterised in that** a first contact surface (12) is provided for the cortex of a resected femoral neck (13) beneath the head (1) medially at the stem (3) and is formed approximately as a triangle when developed; and **in that** a second contact surface (15) is provided for the cortex of the femoral bone (8) for the region of the femoral medullary space (14) laterally at the stem (3) in order to transfer a tilt movement of the stem (3) to the bone (13, 8).

7. A femoral head prosthesis in accordance with claim 6, **characterised in that** the first and second contact surfaces (12, 15) each correspond to an area from 150 mm² to 450 mm².

8. A femoral head prosthesis in accordance with any one of the claims 1 to 7, **characterised in that** the stem (3) has a collar (16) towards the head (1).

9. A femoral head prosthesis in accordance with claim 8, **characterised in that** the collar (16) lies in a plane (18) with which a certain position is associated such that the plane (18) forms an angle with its normal (19) of α = 87° in the projection onto the frontal plane with respect to a horizontal X axis extending towards medial, an angle of β = 15° with the vertical Z axis in the projection onto the sagittal plane and an angle of γ = 13° with the horizontal axis extending towards medial in the projection onto the transversal plane, with the angular figures being able to have a spread of plus/minus 3°.

10. A femoral head prosthesis in accordance with any one of the claims 1 to 9, **characterised in that** the head (1) and the stem (3) consist of different materials.

11. A femoral head prosthesis in accordance with claim 10, **characterised in that** the head (1) and the stem (3) each consist of a non-metallic material.

12. A femoral head prosthesis in accordance with claim 11, **characterised in that** the head (1) consists of a ceramic material.

13. A femoral head prosthesis in accordance with claim 10, **characterised in that** the head (1) is made as a hollow metal sphere into which a stem (3) made of plastic is moulded.

14. A femoral head prosthesis in accordance with claim 10, **characterised in that** the head (1) has an extension (17), to which a stem (3) is moulded.

15. A femoral head prosthesis in accordance with any one of the preceding claims, **characterised in that** the stem (3) is reinforced by fibre material or fillers in order to set the modulus of elasticity to a desired value.

## Revendications

1. Prothèse du col du fémur comportant une tête (1) sphérique, à travers laquelle est défini un centre de sphère (2), et comportant un fût (3) qui s'étend depuis la hauteur du centre de sphère (2) le long d'une ligne médiane (4) sur une longueur L jusqu'à une extrémité distale (5), **caractérisée en ce que** le fût présente un module d'élasticité E entre 10 GPa et 60 GPa, **en ce que** la longueur L est entre 50 mm et 150 mm, et **en ce que** la rigidité équivalente S = E · L formée par le produit du module d'élasticité E et la longueur L est entre 0,5 · 10⁹ et 9 · 10⁹ [Newton/mètre].

2. Prothèse du col du fémur selon la revendication 1, **caractérisée en ce que** la ligne médiane (4) du fût (3) présente une courbure (6).

3. Prothèse du col du fémur selon l'une des revendications 1 ou 2, **caractérisée en ce que** le fût (3) présente une flexion (9) à son extrémité (5) vers le bas dans la direction de l'axe longitudinal (7) d'un os fémoral (8).

4. Prothèse du col du fémur selon l'une des revendications 1 à 3, **caractérisée en ce que** le fût (3) présente perpendiculairement à la ligne médiane (4) jusqu'à son extrémité (5) distale des sections transversales (10) elliptiques dont la surface diminue plus on s'approche du côté distal.

5. Prothèse du col du fémur selon la revendication 4, **caractérisée en ce que** les grands axes (11) des sections transversales elliptiques (10) exécutent avec le déplacement du côté proximal vers le côté distal une rotation d'un angle de rotation ε ≤ 8°.

6. Prothèse du col du fémur selon l'une des revendications 1 à 5, **caractérisée en ce qu'**au-dessous de la tête (1) en situation médiale du fût (3), une première surface de contact (12) réalisée à peu près sous forme de triangle en développement est prévue pour le cortex d'un col du fémur (13) réséqué, et **en ce que** pour la région de l'espace médullaire fémoral (14), il est prévu latéralement sur le fût (3) une deuxième surface de contact (15) pour le cortex de l'os fémoral (8) pour transmettre un couple de basculement du fût (3) à l'os (13, 8).

7. Prothèse du col du fémur selon la revendication 6, **caractérisée en ce que** la première et la deuxième surface de contact (12, 15) correspondent chacune à une surface de 150 mm² à 450 mm².

8. Prothèse du col du fémur selon l'une des revendications 1 à 7, **caractérisée en ce que** le fût (3) présente vers la tête (1) un col (16).

9. Prothèse du col du fémur selon la revendication 8, **caractérisée en ce que** le col (16) est situé dans un plan (18) auquel est associé une position déterminée de telle sorte que le plan (18) forme avec sa normale (19) un angle α = 87° dans la projection sur le plan frontal par rapport à un axe X horizontal s'étendant en direction médiale, un angle β = 15° dans la projection sur le plan sagittal avec l'axe Z vertical et un angle γ = 13° dans la projection sur le plan transversal avec l'axe X horizontal s'étendant en direction médiale, les indications angulaires pouvant dévier de plus/moins 3°.

10. Prothèse du col du fémur selon l'une des revendications 1 à 9, **caractérisée en ce que** la tête (1) et le fût (3) sont en des matériaux différents.

11. Prothèse du col du fémur selon la revendication 10, **caractérisée en ce que** la tête (1) et le fût (3) sont en un matériau non métallique.

12. Prothèse du col du fémur selon la revendication 11, **caractérisée en ce que** la tête (1) est en un matériau céramique.

13. Prothèse du col du fémur selon la revendication 10, **caractérisée en ce que** la tête (1) est réalisée sous forme de sphère métallique creuse dans laquelle est intégré un fût (3) en matière plastique.

14. Prothèse du col du fémur selon la revendication 10, **caractérisée en ce que** la tête (1) présente un prolongement (17) sur lequel est conformé le fût (3).

15. Prothèse du col du fémur selon l'une quelconque des revendications précédentes, **caractérisée en ce que** le fût (3) est renforcé par du matériau en fibres ou par des matières de remplissage pour amener le module E à une valeur souhaitée.
